# EUROPEAN PATENT APPLICATION

(11) **EP 1 658 807 A1**
(43) Date of publication of application: **24.05.2006**
(21) Application number: 05011279.6
(22) Date of filing: 25.05.2005
(51) Int. Cl.: A61B 5/00, A61B 5/02

(54) **Blood pressure measuring device and monitor system with wireless transmission of pressure signals**

(30) Priority: 23.11.2004 CN 200410092695
(71) Applicant: SU, Tsung-Kun, Shijr City, Taipei, Taiwan 221 (TW)
(72) Inventor: SU, Tsung-Kun, Shijr City, Taipei, Taiwan 221 (TW)
(74) Representative: Helms, Joachim

(57) **Abstract**

A blood pressure measuring apparatus with a wireless transmission of pressure signals comprises a tourniquet used for being bound around an arm of a human body and a main body fixedly combined with the tourniquet; the main body can control the expansion and the compression of the tourniquet to process a blood pressure measurement of the human body. Here, the main body has a communication module conformed to a standard communication protocol so as to process a two-way data transmission with another electric product with a wireless communication module conformed to a standard communication protocol, a display device of the another electric product is utilized to show measured values when the blood pressure of the human body is measured.

## Description

### BACKGROUND OF THE INVENTION

### 1. Field of the Invention

The present invention relates to a blood pressure measuring apparatus for a living body, and more particularly to a blood pressure apparatus with a tourniquet and an electric wireless signal transmission module using the tourniquet to combine with an arm of a human body to detect the pulsation of blood vessels to measure a blood pressure and output blood pressure measuring values wirelessly.

### 2. Description of Related Art

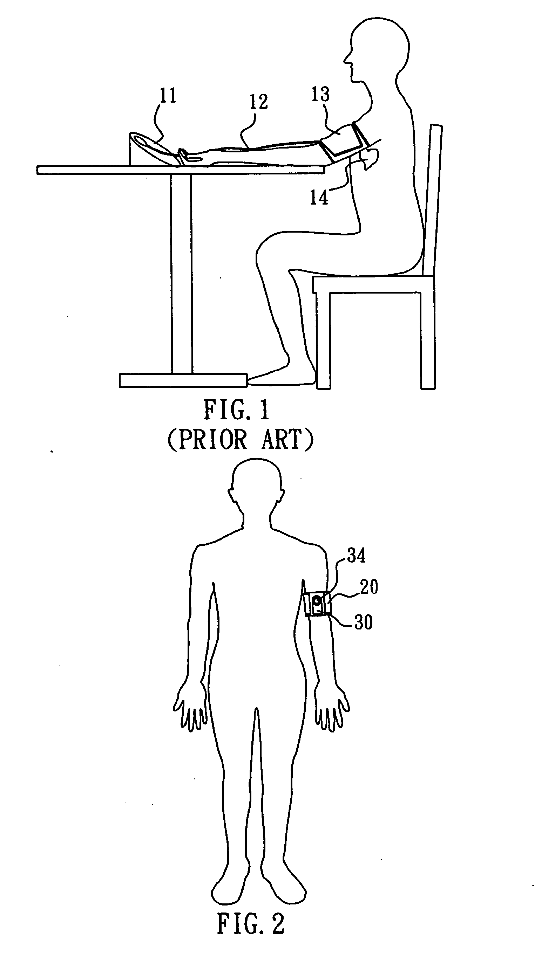

Please refer to FIG. 1. A general electric manometer consists of a main body 11, air tube 12 and tourniquet 13. When the blood pressure of a human body is measured, the tourniquet 13 must be bound around an arm of the human body and the main body 11, the tourniquet 13 must be maintained at the same level height as a heart 14 of the human body so that an rather accurate blood pressure can be measured. Therefore, a table or bed is needed for such kind of the measurement. But, the inconvenience of the measurement tends to cause the measurement of the blood pressure to be inaccurate because an incorrect method is used. Besides, the entire structure of the electric manometer mentioned above is rather heavy such that the measurement of the blood pressure is inconvenient owing to the limitations of the length of the air tube 12 and the position of the level height of the main body 11 and an inaccurate blood pressure could not be measured owing to the carelessness.

Taiwan patent No. 242, 734 discloses a blood pressure automatically measurable electric watch structure, comprising a main body in which electric elements of the electric watch are disposed and watch bands wearable around a wrist are disposed at both ends thereof, a gas-filled bag disposed at the bottom of the main body and wearable around the wrist, a pressure sensor disposed at the bottom of the main body for measuring the blood pressure, a display device disposed on the surface of the main body for showing the time, the blood pressure and the pulse rate, a gas filling pump connected with the gas-filling bag for compressing the air and filling the gas-filling bag therewith, a low speed exhaust valve connected with the gas-filled bag for exhausting the air in the gas-filling bag slowly and a electric power supply disposed in the main body for providing electric power.

The blood pressure automatically measurable electric watch structure disclosed in the Taiwan patent mentioned above is convenient in carrying and measuring the blood pressure. But, a user must notice to place the electric watch to be positioned at the same level height as the heart of the human body and an accurate blood pressure value can then be measured when the blood pressure is measured. Besides, the measured blood pressure cannot be output to other electric devices for further storage or use.

US Pat. NO. 6,344,025 disclosed a blood pressure monitor, comprising a tourniquet for being bound around an arm or wrist of a living body, an air component allowing the tourniquet to be filled in or exhausted of gas and a main body detachably combined with the tourniquet. The main body and the tourniquet both have electric elements for allowing a wired or wireless communication to be existed between them.

The tourniquet of the blood pressure monitor disclosed in the US patent mentioned above has a base seat is used to combine detachably with a main body; there is a whole set electric connection between the tourniquet and the main body. The measurement of the blood pressure of a human body is more convenient when the tourniquet is separated from the main body, but the measured blood pressure values also cannot be output to other electric devices for further storage and application.

### SUMMARY OF THE INVENTION

For further elevating the practical functions of a conventional blood pressure measuring device to allow it to be electrically connected to a general electric product such as a personal computer, notebook computer, personal digital assistant (PDA), cellularphone and set-top-box (STB) and all display device of the electric products are used to displaymeasured values and the measured values are stored to provide a further analytical application, the present invention is proposed.

One object of the present invention is to provide a blood pressure measuring device and monitor system with a wireless transmission of pressure signals, capable of wirelessly transmitting measured values conformed to a standard communication protocol to every kind of electric product with a display device.

Another object of the present invention is to provide a blood pressure measuring device and monitor system with a wireless transmission of pressure signals, capable of utilizing a display device of every kind of electric product to display measured values to reduce resource wasting of repeated dispositions of display devices.

Still another object of the present invention is to provide a blood pressure measuring device and monitor system with a wireless transmission of pressure signals, capable of allowing measuring a blood pressure of a human body regularly, wirelessly transmitting measured values, measuring blood pressure values of a plurality of human bodies at the same time and processing the display of the blood pressure values to allow the management work of the storage and the analysis to be more practicable.

### BRIEF DESCRIPTION OF THE DRAWINGS

The present invention can be more fully understood by reference to the following description and accompanying drawings, in which:
FIG. 1 is a schematic view, showing that a conventional electric manometer is used to measure a blood pressure of a human body;
FIG. 2 is a schematic view, showing that a manometer of the present invention is used to measure a blood pressure of a human body;
FIGS. 3A to 3F are schematic views, showing that a manometer of the present invention and other electric products are used to process wireless transmissions of measured signals;
FIG. 4 is a block diagram, showing a functional structure of a blood pressure measuring device of the present invention and other wireless devices; and
FIG. 5 is a block diagram, showing a functional structure of a blood pressure measuring device of another preferred embodiment according to the present invention,

### DETAILED DESCRIPTION OF THE PREFERRED EMBODIMENTS

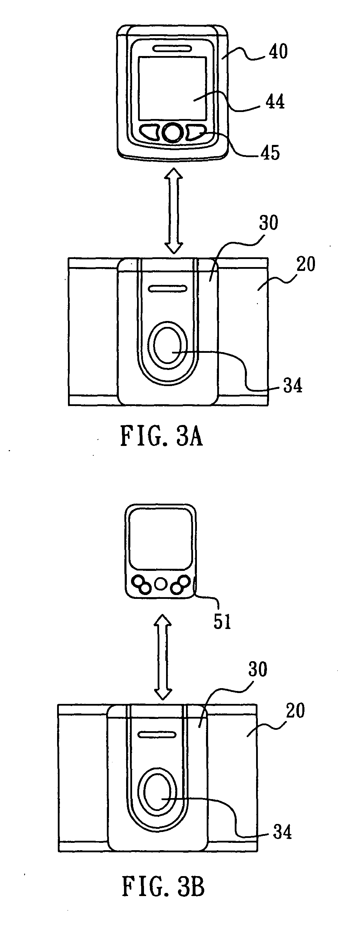

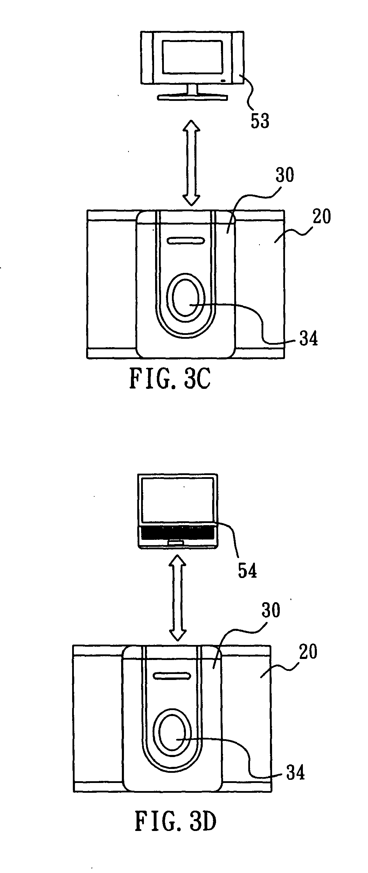

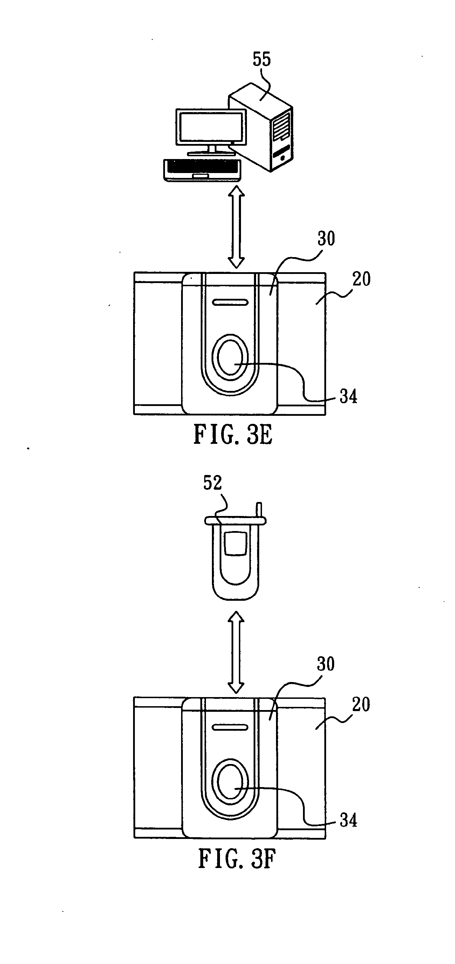

Please refer to FIGS. 2 and 3A to 3F. A blood pressure measuring device with a wireless transmission of pressure signals of a preferred embodiment according to the present invention comprises a tourniquet 20 connected to a main body 30, and the main body 30 mentioned above is better fixed closely on the tourniquet 20 in order to be carried conveniently. The biggest difference between a blood pressure measuring device of the present invention and a conventional blood pressure monitoring device is that the main body 30 of the embodiment of the present invention has a wireless communication module such as Bluetooth and infrared rays conformed to a standard communication protocol; the communication module can be processed a two-way data transmission with any one of electric products such as a signal receiver 40 with a wireless communication module conformed to a standard communication protocol, personal digital assistant 51, cellular phone 52, television set 53 with a set-top-box, notebook computer 54 and personal computer 55. Display devices of the electric products are used to display measured value obtained from the measuring device used to measure a human body and memories of the electric products are provided to store the measured values for further processing analytical applications to allow the measured values to be broadly transmitted to every kind of current common electric product so as to be broadly applied. An other difference between a blood pressure measuring device of the present invention and a conventional blood pressure monitoring device is that a display device needs not to be disposed in the main body 30 of the embodiment according to the present invention and display devices of other electric products can respectively be used to display the measured values obtained from the blood pressure measuring device used to measure a human body so that a resource waste caused from the repeated dispositions of display devices can be reduced. The blood pressure measuring device and monitoring system of the embodiment of the present invention can allow institutes such as a hospital, sanatorium and rest home to process frequent blood pressure measurements and recording for a group of persons. Each main body can be controlled to process the blood pressure measurement of a human body only by connecting a personal computer mainframe wirelessly with the main body combined with the human body and the measured value is then transmitted back to the personal computer mainframe. Whereby, blood pressure managements such as blood pressure measurements, recordings, displays, storages, analyses for a group of persons can be more practicable.

The tourniquet 20 of the blood pressure measuring device of the embodiment according to the present invention can be bound around an arm of a human body as FIG. 2 shows, the level position thereof is at the same height as a heart of the human body so that the blood pressure measurement can be processed any time and is not subjected to the limitation of a location.

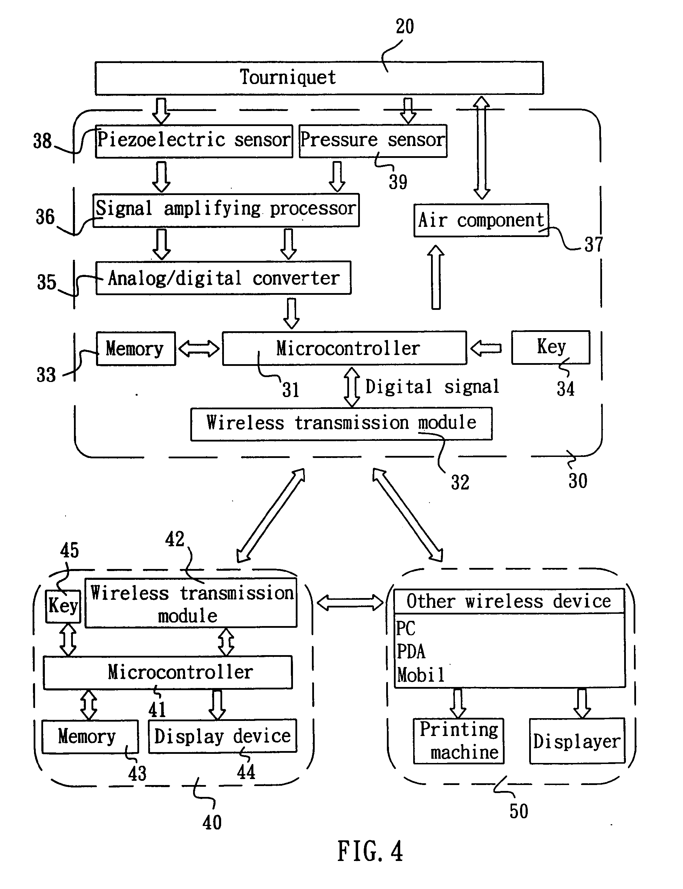

Please refer to FIG. 4. A main body 30 of another preferred embodiment according to the present invention comprises a microcontroller 31 respectively electrically connected with a wireless transmission module 32, memory 33, key 34, analog/digital converter 35, signal amplifying processor 36, air component 37 including a pump and exhaust valve, in which the analog/digital converter 35 is further electrically connected to the signal amplifying processor 36 and the signal amplifying processor 36 is further connected respectively to a piezoelectric sensor 38 and pressure sensor 39. The piezoelectric sensor 38 and the pressure sensor 39 are respectively fixedly combined with the tourniquet 20. And, the air component 37 is communicated with the air in the tourniquet 20 to allow the tourniquet 20 to be expanded or compressed. The air component of the embodiment is formed in the main body, but it can also be formed in the tourniquet.

The main body 30 of the blood pressure measuring device and monitoring system of the embodiment of the present invention can be wirelessly connected with a signal receiver 40 or other wireless devices 50 such as a personal computer, PDA or cellular phone. Here, the signal receiver 40 has a microcontroller 41 electrically connected respectively to a wireless transmission module 42, memory 43, display device 44 and key 45

The microcontroller 31 controls the pump of the air component 37 to fill the tourniquet 20 with air up to a preset pressure after the main body 30 of the present invent receives a control signal for actuating the measurement of the blood pressure of a human being emitted from the signal receiver 40 or other wireless device 50 and then control exhaust valve to exhaust the air slowly. The piezoelectric sensor 38 soon senses the pressure exerted thereon from the blood vessels of the human body and the pressure sensor 39 soon senses the air pressure in the tourniquet 20 at this moment, the sensed signals are respectively transmitted to the signal amplifying processor 36 to be amplified. Thereafter, the signals are transmitted to the analog/digital converter 35 to transform to digital measurement signals. The microcontroller 41 can then control the memory 43 to store the measurement data or the display device 44 to show blood pressure values and pulse numbers after the signal receiver 40 received the digital measurement signals. The similar process can also be carried out after another wireless device 50 receives the digital measure value, the measure blood pressure values and pulse numbers are printed by a printing machine, or the measured data are shown on the displayer.

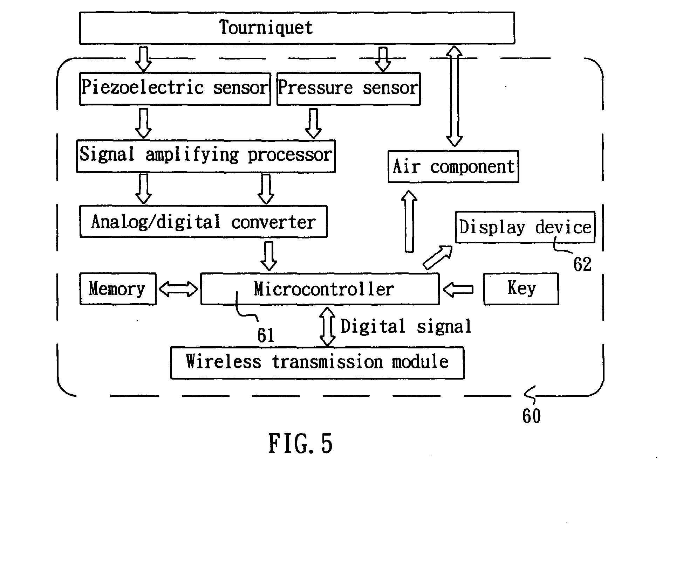

Although a display device is not included in the main body 30 of the blood pressure measuring device of the embodiment mentioned above, the present invention is not limited to this; the display device can be facilitated in a main body of another embodiment according to the present invention. As FIG. 5 shows, a main body 60 of another preferred embodiment according to the present invention has a microcontroller 61 further connected to a display device 62. Furthermore, the electric product used to receive the pressure signal transmitted from the main body is mot limited toa signal receiver, PDA, cellularphone, television connect with a set-top-box, notebook computer or personal computer mentioned above; any electric product with a wireless communication module conformed to the standard communication protocol and a display device can be suited to the present invention.

A control signal for processing a blood pressure measurement regularly or immediately is emitted through the keys of other wireless devices 50 or the key 45 of the receiver 40. After the microcontroller 31 of the main body 30 detects the control signal through the wireless transmission module 32, it then controls the memory 33 to store the data of the regularly processed blood pressure measurement, actuate the air component 37 to fill and exhaust air into and out of the tourniquet 20 depending on a preset time or a control signal for processing a blood pressure measurement immediately to process the blood pressure measurement and control the wireless transmission module 32 to process the wireless transmission of the measured values. A person whose blood pressure is measured can control the main body 30 to stop the blood pressure measurement operation by pressing the key 34 down.

When the tourniquet of the present invention is bound around pulse point of a human being' s arm, that the heart, the pulse and the blood pressure measurement device at a same level height can be attained not matter what he stands, sits or lays down.

That the blood pressure measurement values are not correct so as to lead to a misjudgment because the limitation of the connection and length of the tube to influences a measurement posture and the posture caused from the observation of the measurement display is improper when the blood pressure is measured can be improved according to the present invention.

Additional advantages and modifications will readily occur to those skilled in the art. Therefore, the invention in its broader aspects is not limited to the specific details and representative embodiments shown and described herein. Accordingly, various modifications may be made without departing from the spirit or scope of the general inventive concept as defined by the appended claims and their equivalents.

## Claims

1. A blood pressure measuring apparatus with a wireless transmission of pressure signals, comprising:
a tourniquet, used for being bound around an arm of a human body;
a main body, fixedly combined with said tourniquet, an air component communicated with air in said tourniquet being disposed in said main body for controlling the expansion and the compression of said tourniquet to process a blood pressure measurement of the human body, said main body having a communication module conformed to a standard communication protocol so as to process a two-way data transmission with another electric product with a wireless communication module conformed to a standard communication protocol, a display device of said another electric product being utilized to show measured values when the blood pressure of the human body is measured.

2. The blood pressure measurement device according to claim 1, wherein said wireless communication module is a Bluetooth communication module.

3. The blood pressure measurement device according to claim 1, wherein said wireless communication module is infrared wireless communication module.

4. The blood pressure measurement device according to claim 1, wherein said main body comprises a microcontroller respectively electrically connected with a wireless transmission module, analog/digital converter, signal amplifying processor and air component comprising a pump and exhaust valve, in which said analogy/digital converter is further electrically connected to a signal amplifying processor, said signal amplifying processor is further respectively connected to a piezoelectric sensor and pressure sensor, in which said piezoelectric sensor and said pressure sensor are respectively fixedly combined with said tourniquet; whereby, when said piezoelectric sensor senses the pressure exerted by blood vessels of a human body, said pressure sensor senses the air pressure in said tourniquet and the sensed signals are respectively electrically transmitted to said signal amplifying processor to do an amplification process, and thereafter transmitted to said analog/digital converter to convert into digital measurement signals, said microcontroller detects said digital measurement signals and then controls said wireless transmission module to output said digital measurement signals.

5. The blood pressure measurement device according to claim 4, wherein said controller is further electrically connected to a key; whereby, said main body is allowed to control to stop the measurement to the blood pressure of the human body when said microcontroller detects signals emitted from said key.

6. The blood pressure measurement device according to claim 4, wherein said microcontroller is further electrically connected to a memory so as to store regular times for the measurement of the blood pressure of the human body.

7. The blood pressure measurement device according to claim 4, further comprising a signal receiver, said signal receiver having a microcontroller respectively electrically connected with a wireless transmission module, memory and display device; whereby, said signal receiver is wirelessly connected to said main body and said microcontroller then controls aid memory to store said measurement data or said display device to display said measurement of blood pressure values and pulse numbers.

8. The blood pressure measurement device according to claim 7, wherein said microcontroller of said signal receiver is further connected to at least one key; whereby, a user utilizes said key(S) to emit a control signal to control said main body to process a regular or immediate the measurement of the blood pressure of the human body.

9. The blood pressure measurement device according to claim 1, wherein said main body further comprises a display device.

10. A blood pressure monitoring system, comprising:
a tourniquet, used for binding around a human body;
a main body, fixedly connected to said tourniquet for process a blood pressure measurement of the human body, said main body having a wireless communication module conformed to a standard communication protocol;
an electric product with a displace device, being processed a two-way data transmission with said main body, said displaying measured values obtained from the measurement of the blood pressure measurement of the human body;
wherein said main body comprises a microcontroller respectively electrically connected with a wireless transmission module, analog/digital converter, signal amplifying processor and air component comprising a pump and exhaust valve, in which said analogy/digital converter is further electrically connected to a signal amplifying processor, said signal amplifying processor is further respectively connected to a piezoelectric sensor and pressure sensor, in which said piezoelectric sensor and said pressure sensor are respectively fixedly combined with said tourniquet; whereby, when said piezoelectric sensor senses the pressure exerted by blood vessels of a human body, said pressure sensor senses the air pressure in said tourniquet and the sensed signals are respectively electrically transmitted to said signal amplifying processor to do an amplification process, and thereafter transmitted to said analog/digital converter to convert into digital measurement signals, said microcontroller detects saiddigitalmeasurement signals and then controls said wireless transmission module to output said digital measurement signals.
